# EUROPEAN PATENT APPLICATION

(11) **EP 3 971 568 A1**
(43) Date of publication of application: **23.03.2022**
(21) Application number: 20806856.9
(22) Date of filing: 23.04.2020
(51) Int. Cl.: G01N 33/543

(54) **CARRIER FILM AND INSPECTION KIT**

(30) Priority: 15.05.2019 JP 2019092042
(71) Applicant: Denka Company Limited, Tokyo 103-8338 (JP)
(72) Inventor: AOYAMA, Shuhei, Tokyo 103-8338 (JP); AKIYAMA, Yuto, Tokyo 103-8338 (JP)
(74) Representative: Thomann, William John
(86) International application number: PCT/JP2020/017476
(87) International publication number: WO 2020/230572

(57) **Abstract**

In one cross-section, a protrusion (8) has a first region (RG1) . An outer edge of the first region (RG1) of the protrusion (8) has a concave portion (R). The concave portion (R) is recessed in an X direction by a distance D with respect to a virtual straight line (IL). The virtual straight line (IL) extends in a Y direction. Specifically, the virtual straight line (IL) is in contact with a first position (P1) of an outer edge of the protrusion (8) and passes through a second position (P2).

## Description

### TECHNICAL FIELD

The present invention relates to a membrane carrier and a test kit.

### BACKGROUND ART

In recent years, a Point of Care Test (POCT, clinical site real-time testing) reagent, which measures an affliction of an infectious disease, pregnancy, a blood glucose level, and the like by using an antigen-antibody reaction and the like, has attracted attention. The POCT reagent is, for example, a reagent for a test performed by a subject or a test performed by the subject himself/herself, and has characteristics that a result can be determined in a short time, a use method is simple, and the cost is low. With these characteristics, the POCT reagent is frequently used for medical examinations and periodic medical examinations at a stage where the symptoms are mild, and is an important medical examination tool in home medical care, which is expected to increase in the future.

In many POCT reagents, a determination is made by introducing a liquid sample such as blood into a test kit and detecting a specific substance to be detected contained therein. As a method for detecting a specific substance to be detected from the liquid sample, an immunochromatography method is often used. An immunochromatography method refers to a technique in which a substance to be detected and a labeling substance are bound while a liquid dropped on a membrane carrier for a test kit moves on the membrane carrier, these specifically bind to a substance (hereinafter, referred to as detection substance) immobilized in the test kit, and a change or the like in color or mass generated as a result is detected. The detection substance may be referred to as a reagent.

A nitrocellulose membrane is often used as a membrane carrier for moving a liquid sample (Patent Document 1). The nitrocellulose membrane has a large number of fine holes having a diameter of about several µm, and the liquid sample moves in the holes by capillary force.

However, since the nitrocellulose membrane is derived from a natural product, and a hole diameter and a way of connecting the holes are not uniform, there occurs a difference in a flow rate of the liquid sample flowing in each membrane. In a case where a difference occurs in the flow rates, the time required to detect a substance to be detected is also changed, and as a result, the substance to be detected may be erroneously determined as non-detection before binding occurs.

In order to solve the above-mentioned problem, a liquid sample test kit of artificially preparing a fine flow path has been devised (Patent Document 2). In Patent Document 2, by using a synthetic material, it is possible to prepare a membrane carrier having a uniform structure. Therefore, it is possible to decrease a possibility of erroneously determining a substance to be detected as non-detection before binding occurs.

In a case where a synthetic material is used, it is necessary to increase the affinity between the detection substance and the material in order to improve the detection sensitivity, and it is considered effective to perform various surface treatments on the material in advance (Patent Documents 3 and 4). Patent Document 5 discloses a membrane carrier for a liquid sample test kit for detecting a substance to be detected in a liquid sample, in which at least one flow path capable of transporting the liquid sample is provided, and the microstructure that generates a capillary action for transporting the liquid sample is provided on a bottom surface of the flow path.

### RELATED DOCUMENT

### PATENT DOCUMENT

[Patent Document 1] Japanese Unexamined Patent Publication No. 2014-062820
[Patent Document 2] Japanese Patent No. 5799395
[Patent Document 3] Japanese Unexamined Patent Publication No. 2013-113633
[Patent Document 4] US Patent Application Publication No. 2011/0284110
[Patent Document 5] International Publication No. WO

### SUMMARY OF THE INVENTION

### TECHNICAL PROBLEM

Further improvement in detection sensitivity is required for the above-mentioned test kits.

The present invention has been made in view of the above circumstances, and an object of the present invention is to provide a membrane carrier capable of obtaining a test kit with improved detection sensitivity and a test kit with improved detection sensitivity.

### SOLUTION TO PROBLEM

The present inventors have made extensive studies to achieve the above object. As a result, it was found that by an outer edge of a protrusion formed in a detection zone of the membrane carrier having a specific shape, the detection sensitivity of the substance to be detected in the liquid sample can be improved, thereby achieving the present invention.

That is, according to the present invention, a membrane carrier and a test kit are provided as follows.

[1] A membrane carrier including: a detection zone having a first surface on which a protrusion is formed,
   in which in one cross-section, the protrusion includes a first region positioned on one side of the protrusion from a center of the protrusion in a width direction of the protrusion, and a second region positioned on other side of the protrusion from the center of the protrusion in the width direction of the protrusion, and
   in the one cross-section, at least one of an outer edge of the first region and an outer edge of the second region of the protrusion has a concave portion.
[2] The membrane carrier according to [1],
   in which in the one cross-section, the at least one of the outer edge of the first region and the outer edge of the second region of the protrusion has a plurality of concave portions recessed by equal to or more than 50 nm and equal to or less than 500 nm in a direction parallel to the first surface with respect to a virtual straight line being in contact with a first position of the outer edge and passing through a second position of the outer edge positioned below the first position in a direction perpendicular to the first surface.
[3] The membrane carrier according to [1] or [2],
   in which an arithmetic mean roughness Ra1 of at least a part of the outer edge of the protrusion in the first region calculated by a method including the following steps or an arithmetic mean roughness Ra2 of at least a part of the outer edge of the protrusion in the second region calculated by the method including the following steps is equal to or more than 0.020 µm and equal to or less than 1.000 µm.
   (Step 1) Extracting a profile of the at least a part of the outer edge
   (Step 2) Trend removing the profile so that a start point and an end point of the profile overlap with a fitting curve of the profile
   (Step 3) Calculating Ra1 or Ra2 by applying 1 µm based on a cutoff value λc defined in JIS B0601: 2013, without applying a cutoff value λs defined in JIS B0601: 2013, to the trend-removed profile
[4] The membrane carrier according to any one of [1] to [3],
   in which a root mean square roughness Rq1 of at least a part of the outer edge in the first region of the protrusion calculated by a method including the following steps or a root mean square roughness Rq2 of at least a part of the outer edge in the second region of the protrusion calculated by the method including the following steps are equal to or more than 0.030 µm and equal to or less than 1.000 µm.
   (Step 1) Extracting a profile of the at least a part of the outer edge
   (Step 2) Trend removing the profile so that a start point and an end point of the profile overlap with a fitting curve of the profile
   (Step 3) Calculating Rq1 or Rq2 by applying 1 µm based on a cutoff value λc defined in JIS B0601: 2013, without applying a cutoff value λs defined in JIS B0601: 2013, to the trend-removed profile
[5]
   The membrane carrier according to any one of [1] to [4], in which the first surface has a plurality of protrusions regularly or translationally symmetrically arranged and including the protrusion.
[6] The membrane carrier according to [5],
   in which a surface roughness of the first surface between adjacent protrusions is smaller than a surface roughness of a surface of the protrusion.
[7] The membrane carrier according to [5] or [6],
   in which a closest contact distance between adjacent protrusions is equal to or more than 0 and equal to or less than 500 µm.
[8] The membrane carrier according to any one of [1] to [7],
   in which the protrusion includes a thermoplastic resin.
[9] The membrane carrier according to any one of [1] to [8],
   in which a surfactant is attached to the protrusion.
[10] A test kit including:
   the membrane carrier according to any one of [1] to [9].

### ADVANTAGEOUS EFFECTS OF INVENTION

According to the present invention, it is possible to provide a membrane carrier capable of obtaining a test kit with improved detection sensitivity and a test kit with improved detection sensitivity.

### BRIEF DESCRIPTION OF THE DRAWINGS

Fig. 1 is an example of an embodiment according to the present invention, and is a schematic top view of a test kit.
Fig. 2 is an example of an embodiment according to the present invention, and is a schematic top view of a membrane carrier.
Fig. 3 (a) is an example of an embodiment according to the present invention, and is a bird's-eye view (top view) of a rough structure A, and Fig. 3(b) is a perspective view of a protrusion constituting the rough structure A shown in Fig. 3(a).
Fig. 4 (a) is an example of an embodiment according to the present invention, and is a bird's-eye view (top view) of the rough structure A, and Fig. 4(b) is a perspective view of a protrusion constituting the rough structure A shown in Fig. 4(a).
Fig. 5 (a) is an example of an embodiment according to the present invention, and is a bird's-eye view (top view) of the rough structure A, and Fig. 5(b) is a perspective view of a protrusion constituting the rough structure A shown in Fig. 5(a).
Fig. 6 (a) is an example of an embodiment according to the present invention, and is a bird's-eye view (top view) of the rough structure A, and Fig. 6(b) is a perspective view of a protrusion constituting the rough structure A shown in Fig. 6(a).
Fig. 7 is a bird's-eye view (top view) of an example of the rough structure A in a case where a distance between the protrusions is 0.
Fig. 8(a) is a view for explaining a first example of a method of measuring various parameters of a protrusion on which a rough microstructure B is formed in the rough structure A, and Fig. 8(b) is a view for explaining a second example of a method of measuring various parameters of the protrusion on which the rough microstructure B is formed in the rough structure A.
Fig. 9 is a diagram for explaining a concave portion of the outer edge in the first region of the protrusion.
Fig. 10 is an example of an embodiment according to the present invention, and is a schematic view of surface treatment.
Fig. 11(a) is a diagram showing an electron micrograph showing an enlarged view of a protrusion according to Example 1, and Fig. 11 (b) is an electron micrograph showing an enlarged view of a protrusion according to Example 2.

### DESCRIPTION OF EMBODIMENTS

Hereinafter, embodiments of the present invention will be described with reference to the drawings. In addition, the drawings are schematic views, and do not match the actual dimensional ratio.

### 1. Membrane carrier

The membrane carrier for a test kit according to the present embodiment (also simply referred to as "membrane carrier") is a membrane carrier for a test kit for detecting a substance to be detected in a liquid sample, can transport the liquid sample, and has a flow path having a detection zone, in which the flow path can generate a capillary action for transporting the liquid sample, and has a rough structure A having a protrusion.

Then, in the membrane carrier for a test kit according to the present embodiment, a rough microstructure B is formed on a surface of the protrusion at least in the detection zone. The membrane carrier includes a detection zone having a first surface (a surface including a flat portion) on which the protrusion is formed. In one cross-section, the protrusion has a first region and a second region. The first region of the protrusion is positioned on one side of the protrusion from the center of the protrusion in a width direction of the protrusion. The second region of the protrusion is positioned on the other side of the protrusion from the center of the protrusion in the width direction of the protrusion. In the one cross-section, at least one of an outer edge of the first region and an outer edge of the second region of the protrusion has a concave portion. Specifically, in the one cross-section, at least one of the outer edge of the first region and the outer edge of the second region of the protrusion has a plurality of concave portions recessed by equal to or more than 50 nm and equal to or less than 500 nm in a direction parallel to the first surface with respect to a predetermined virtual straight line. The number of the concave portions is, for example, equal to or more than three. The predetermined virtual straight line is in contact with the first position of the outer edge and passes through the second position of the outer edge positioned below the first position in the direction perpendicular to the first surface. Here, in a case where a concave portion having a depth of equal to or more than 1.0 µm is formed at a tip of the protrusion in the one cross-section, the first region and the second region are regions excluding the concave portion.

A silane coupling agent may be attached to a surface of the rough microstructure B.

In addition, the rough microstructure B formed on the surface of the protrusion according to the present embodiment has a macroscopic shape such as a striped shape or a streak shape.

In the membrane carrier for a test kit according to the present embodiment, as the number of the concave portions in the first region or the second region is equal to or more than a certain number, it is possible to increase the amount of the detection substance carried in the detection zone. As a result, the detection sensitivity of the substance to be detected in the liquid sample can be improved.

The reason why the amount of the detection substance carried in the detection zone can be increased is not clear, but the following reasons are considered.

First, the matter that the number of the concave portions in the first region or the second region is equal to or more than a certain number is an index indicating that the surface area of the protrusions is increased due to the presence of the rough microstructure B.

Therefore, at least in the detection zone, the rough microstructure B in which the number of the concave portions in the first region or the second region is equal to or more than a certain number is considered to have a structure having a space suitable for carrying the detection substance. Therefore, since the membrane carrier for a test kit according to the present embodiment has a rough microstructure B in which the number of concave portions in the first region or the second region at least in the detection zone is equal to or more than a certain number, it is considered that it is possible to increase the amount of the detection substance carried in the detection zone.

However, it is considered that the rough microstructure B related to the improvement of the detection sensitivity is a rough microstructure B formed on a portion of the surface of the protrusion in which a macroscopically large concave portion is not present. It is considered that this is because the macroscopically large concave portion has a structure having a space not suitable for carrying the detection substance. There is a case where a macroscopically large concave portion is formed at the tip of the protrusion. In this case, the first region and the second region should be regions excluding the region where the macroscopically large concave portion is formed.

From the above, it is considered that the membrane carrier for a test kit according to the present embodiment can improve the detection sensitivity of the substance to be detected in the liquid sample as the number of the concave portions in the first region or the second region is a certain number.

Due to the rough microstructure B, a surface roughness of the protrusion becomes large.

In one example, the arithmetic mean roughness Ra1 of at least a part of the outer edge of the protrusion in the first region calculated by a method including the following steps or the arithmetic mean roughness Ra2 of at least a part of the outer edge of the protrusion in the second region calculated by a method including the following steps can be equal to or more than 0.020 µm and equal to or less than 1.000 µm.
(Step 1) Extracting a profile of at least a part of the outer edge
(Step 2) Trend removing the profile so that a start point and an end point of the profile overlap with a fitting curve of the profile
(Step 3) Calculating Ra1 or Ra2 by applying 1 µm based on a cutoff value λc defined in JIS B0601: 2013, without applying a cutoff value λs defined in JIS B0601: 2013, to the trend-removed profile

In one example, the root mean square roughness Rq1 of at least a part of the outer edge of the protrusion in the first region calculated by a method including the following steps or the root mean square roughness Rq2 of at least a part of the outer edge of the protrusion in the second region calculated by a method including the following steps can be equal to or more than 0.030 µm and equal to or less than 1.000 µm.
(Step 1) Extracting a profile of at least a part of the outer edge
(Step 2) Trend removing the profile so that a start point and an end point of the profile overlap with a fitting curve of the profile
(Step 3) Calculating Rq1 or Rq2 by applying 1 µm based on a cutoff value λc defined in JIS B0601: 2013, without applying a cutoff value λs defined in JIS B0601: 2013, to the trend-removed profile

In the membrane carrier for a test kit according to the present embodiment, a surfactant may be attached to the protrusions, particularly to the surface of the rough microstructure B. Here, the surfactant may be physically adsorbed on the surface of the rough microstructure B, may be chemically adsorbed, or may be directly bound to a functional group present on the surface of the rough microstructure B. Even if the membrane carrier exhibits water repellency due to the rough structure A and the rough microstructure B, the water repellency can be reduced by the surfactant, and an amount of the detection substance carried in the detection zone (particularly, amount of liquid) can be maintained.

Examples of the surfactant according to the present embodiment include, but not limited to, sodium cholic acid, sodium deoxycholate, sodium lauryl sulfate, sucrose monocholate; β-D-fractopyranosyl-α-D-glucopyranoside monododecanoate, β-D-fractopyranosyl-α-D-glucopyranoside monodecanoate; n-octanoyl-N-methylglucamide, n-nonanoyl-N-methylglucamide, n-decanoyl-N-methylglucamide, n-octyl-β-D-thioglucopyranoside, n-octyl-β-D-maltopyranoside, n-octyl-β-D-glucopyranoside, n-nonyl-β-D-thiomaltopyranoside, n-dodecyl-β-D-maltopyranoside, n-decyl-β-D-maltopyranoside; N, N-bis (3-D-gluconamide propyl) coramide, N, N-bis (3-D-gluconamide propyl) deoxycholamide; 3-[(3-colamidpropyl) dimethylammonio] propane sulfonic acid, 3-[(3-colamidpropyl) dimethylammonio]-2-hydroxypropane sulfonic acid; ZWITTERGENT 3-10 Detergent (product name) manufactured by Calbiochem, Zwittergent 3-12 Detergent (product name) manufactured by Calbiochem, Zwittergent 3-14 Detergent (product name) manufactured by Calbiochem; Triton X-100 (product name): Polyethylene glycol mono-p-isooctylphenyl ether (Nacalai Tesque Co., Ltd.), Tween 20: Polyoxyethylene sorbitan monolaurate (Nacalai Tesque Co., Ltd.), Tween80: Polyoxyethylene sorbitan mono oleate (Nacalai Tesque Co., Ltd.), NP-40: Nonidet P-40 (Nacalai Tesque Co., Ltd.), Zwittergent: Zwittergent 3-14 (Calbiochem Co., Ltd.), SDS: Sodium dodecyl sulfate (Nacalai Tesque Co., Ltd.), CHAPS: 3-[(3-colamidpropyl) dimethylammonio] propane sulfonic acid (Dojindo), and the like; or those obtained by combining or mixing two or more thereof.

In the membrane carrier for a test kit according to the present embodiment, a silane coupling agent may be attached to the surface of the rough microstructure B. Here, the silane coupling agent may be physically adsorbed on the surface of the rough microstructure B, may be chemically adsorbed, or may be directly bound on a functional group present on the surface of the rough microstructure B.

Examples of the silane coupling agent according to the present embodiment include a silane coupling agent having an amino group, a silane coupling agent having a mercapto group, a silane coupling agent having an epoxy group, a silane coupling agent having an acrylic group, a silane coupling agent having a methacryl group, a silane coupling agent having a vinyl group, and a silane coupling agent having an isocyanate group.

Examples of the silane coupling agent having an amino group include 3-aminopropyltriethoxysilane, 3-aminopropyltrimethoxysilane, 3-(2-aminoethyl) aminopropyltriethoxysilane, 3-(2-aminoethyl) aminopropyltrimethoxysilane, and the like.

Examples of the silane coupling agent having a mercapto group include 3-mercaptopropyltrimethoxysilane, 3-mercaptopropyltriethoxysilane, 2-mercaptoethyltrimethoxysilane, 2-mercaptoethyltriethoxysilane, and the like.

Examples of the silane coupling agent having an epoxy group include 3-glycidoxypropyltrimethoxysilane, 5,6-epoxyhexyltriethoxysilane, 2-(3,4-epoxycyclohexyl) ethyltrimethoxysilane, 3-glycidoxypropyltriethoxysilane, and the like.

Examples of the silane coupling agent having an acrylic group include 3-acryloxypropyltrimethoxysilane, 3-acryloxypropylmethyldimethoxysilane, 3-acryloxypropylmethyldiethoxysilane, 3-acryloxypropyltriethoxysilane, and the like.

Examples of the silane coupling agent having a methacryl group include 3-methacryloxypropylmethyldimethoxysilane, 3-methacryloxypropyltrimethoxysilane, 3-methacryloxypropylmethyldiethoxysilane, 3-methacryloxypropyltriethoxysilane, γ-(methacryloyloxypropyl) trimethoxysilane, γ-(methacryloyloxypropylmethyl) dimethoxysilane, and the like.

Examples of the silane coupling agent having a vinyl group include vinyltriethoxysilane, vinyltrimethoxysilane, and the like.

Examples of the silane coupling agent having an isocyanate group include trimethoxysilylmethyl isocyanate, triethoxysilylmethyl isocyanate, tripropoxysilylmethyl isocyanate, 2-trimethoxysilylethyl isocyanate, 2-triethoxysilylethyl isocyanate, 2-tripropoxysilylethyl isocyanate, 3-trimethoxysilylpropyl isocyanate, 3-triethoxysilylpropyl isocyanate, 3-tripropoxysilylpropyl isocyanate, 4-trimethoxysilylbutylisocyanate, 4-triethoxysilylbutylisocyanate, 4-tripropoxysilylbutylisocyanate, and the like.

These silane coupling agents may be used alone or in combination of two or more.

Among these, at least one selected from a silane coupling agent having an amino group and a silane coupling agent having an epoxy group is preferable from a viewpoint of capable of further increasing the amount of the detection substance carried in the detection zone.

An attached amount of the silane coupling agent is, for example, in a range of equal to or more than 0.1 mg and equal to or less than 10 g per 1 m² of the surface area of a membrane carrier 3.

In the membrane carrier for a test kit according to the present embodiment, it is preferable that a cross-linking agent (crosslinker) is further attached to the surface of the rough microstructure B. By further attaching the cross-linking agent to the surface of the rough microstructure B, the detection substance can be stably carried on the surface of the rough microstructure B. With this, it is possible to further increase the amount of the detection substance carried in the detection zone.

In addition, by using the cross-linking agent, it becomes possible to carry a detection substance that cannot be well adsorbed on the surface of the rough microstructure B due to steric hindrance or the like, and thus it is possible to further increase the amount of the detection substance carried.

Here, the cross-linking agent may be physically adsorbed on the surface of the rough microstructure B, may be chemically adsorbed, or may be directly bonded to the functional group existing on the surface of the rough microstructure B. In addition, the cross-linking agent may be physically adsorbed on the surface of a layer containing the silane coupling agent, may be chemically adsorbed, or may be directly bonded to the silane coupling agent.

Examples of the cross-linking agent according to the present embodiment include formaldehyde, glutaraldehyde, dextran, 1,4-phenyldiisocyanate, toluene-2,4 diisocyanate, polyethyleneimine, hexamethylene diisocyanate, hexamethylene diisothiocyanate, N, N'-polymethylene bisiodoacetamide, N, N'-ethylene bismaleimide, ethylene glycol bissuccinimidyl succinate, bisdiazobenzidine, 1-ethyl-3-(3-dimethylaminopropyl) carbodiimide, succinimidyl 3-(2-pyridyldithio) propionate, N-succinimidyl 4-(N-maleimide methyl) cyclohexane-1-carboxylate, N-hydroxysucciimide, N-sulfosuccinimidyl 4-(N-maleimide methyl) cyclohexane-1-carboxylate, N-succinimidyl (4-iodoacetyl) aminobenzoate, N-succinimidyl 4-(1-maleimidephenyl) butyrate, N-(ε-maleimide caproyloxy) succinimide, iminothiolane, S-acetylmercaptosuccinic anhydride, methyl-3-(4'-dithiopyridyl) propion imidate, methyl-4-mercaptobutyryl imidate, methyl-3-mercaptopropion imidate, N-succinimidyl-S-acetyl mercaptoacetate, and the like.

These cross-linking agents may be used alone or in combination of two or more.

Among these, at least one cross-linking agent selected from glutaraldehyde, dextran, 1,4-phenyldiisocyanate, toluene-2,4 diisocyanate, polyethyleneimine, 1-ethyl-3-(3-dimethylaminopropyl) carbodiimide, N-succinimidyl 4-(N-maleimide methyl) cyclohexane-1-carboxylate, and N-hydroxysucciimide is preferable from a viewpoint of capable of further increasing the amount of the detection substance carried in the detection zone.

The amount of the cross-linking agent attached is, for example, in a range of equal to or more than 0.1 mg and equal to or less than 10 g per 1 m² of the surface area of the membrane carrier 3.

Here, the substance to be detected is not limited at all, and may be any substance that can have an antigen-antibody reaction with an antibody, such as various pathogens and various clinical markers. Specific examples of the substance to be detected include, but not limited to, virus antigens such as influenza virus, norovirus, adenovirus, RS virus, HAV, HBs, and HIV, bacterial antigens such as MRSA, group A streptococcus, group B streptococcus, and genus Legionella bacteria, toxins produced by bacteria, hormones such as mycoplasma, chlamydia trachomatis, and human chorionic gonadotropin, C-reactive protein, myoglobin, cardiac troponin, various tumor markers, pesticides, and environmental hormones. In particular, the substance to be detected is particularly useful for a case of requiring detection and urgent treatment such as influenza virus, norovirus, C-reactive protein, myoglobin, and cardiac troponin. The substance to be detected may be an antigen capable of inducing an immune reaction alone, or a hapten not capable of inducing an immune reaction by itself but capable of binding to an antibody by an antigen-antibody reaction. The substance to be detected is usually in a state of being floated or dissolved in the liquid sample. The liquid sample may be a sample in which the substance to be detected is floated or dissolved in a buffer solution, for example.

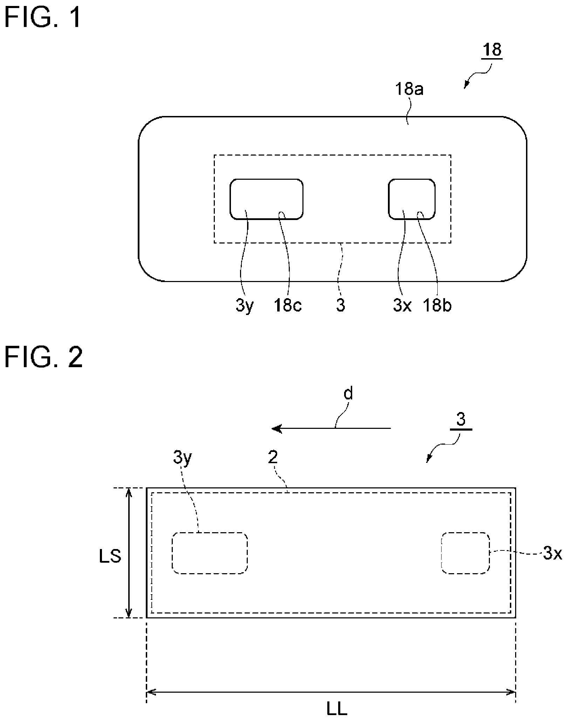

A liquid sample test kit according to the present embodiment (hereinafter, also simply referred to as "test kit 18") has the membrane carrier for a test kit according to the present embodiment and detects a substance to be detected in the liquid sample. Fig. 1 is a schematic top view of the test kit. For example, as shown in Fig. 1, the test kit 18 includes a membrane carrier 3 and a housing 18a that houses the membrane carrier 3. The membrane carrier 3 has, on a surface thereof, a dropping zone 3x in which a liquid sample is dropped, and a detection zone 3y for detecting the substance to be detected in the liquid sample. The dropping zone 3x is exposed at a first opening 18b of the housing 18a. The detection zone 3y is exposed at a second opening 18c of the housing 18a.

Fig. 2 is a schematic top view of the membrane carrier 3. As shown in Fig. 2, the membrane carrier 3 includes at least one flow path 2 that transports a liquid sample. A rough structure A is provided on a bottom surface of the flow path 2 (not shown, details will be described later). The rough structure A is positioned at least between the dropping zone 3x and the detection zone 3y. The rough structure A may be provided over the entire surface of the membrane carrier 3. The entire surface of the membrane carrier 3 may be the flow path 2 of the liquid sample. The rough structure A causes capillary action. Due to the capillary action of the rough structure A, the liquid sample is transported to the detection zone 3y (along a transporting direction d) from the dropping zone 3x through the rough structure A. In a case where the substance to be detected in the liquid sample is detected in the detection zone 3y, color of the detection zone 3y changes.

The entire shape of the membrane carrier 3 is not particularly limited, but may be, for example, a polygon such as a square, a circle, or an ellipse. In a case where the membrane carrier 3 is a square, a vertical width (length in a short-side direction) LS of the membrane carrier 3 may be equal to or more than 1 mm and equal to or less than 100 mm, for example, and a horizontal width (length in a long-side direction) LL of the membrane carrier 3 may be equal to or more than 1 mm and equal to or less than 100 mm, for example. A thickness of the membrane carrier excluding a height of the rough structure A may be equal to or more than 0.1 mm and equal to or less than 10 mm, for example.

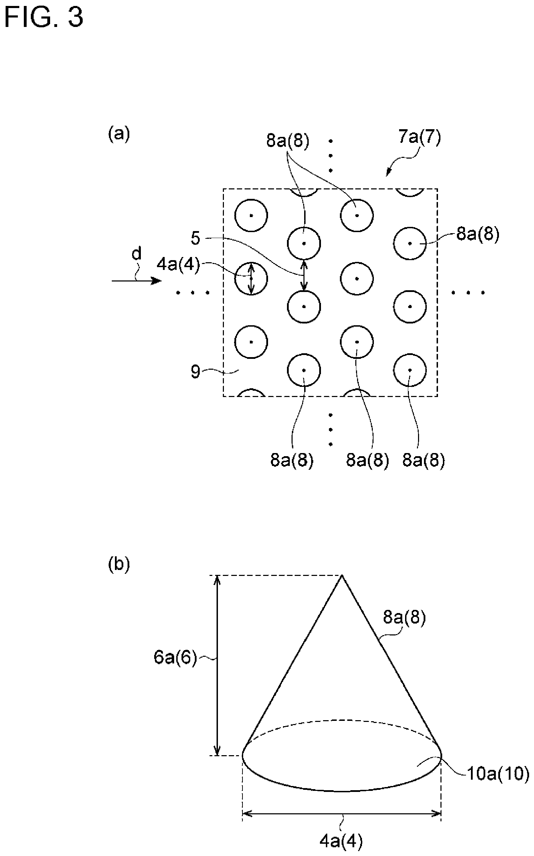

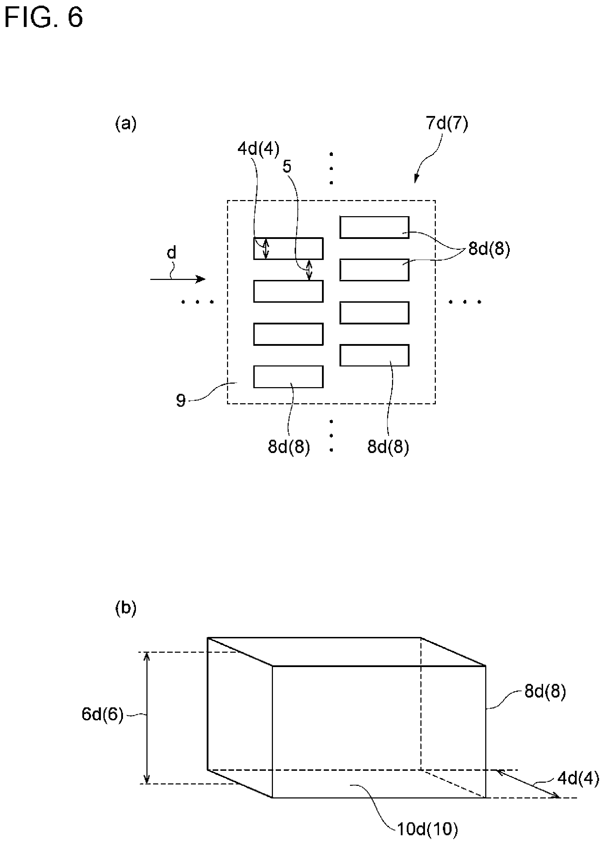

Figs. 3(a) to 6(b) each show an example of a rough structure A provided on a bottom surface of the flow path and a protrusion (also referred to as convex portion) constituting thereof in the present embodiment. In Figs. 3(a) to 6(b), Figs. 3(a), 4(a), 5(a), and 6(a) each are a bird's-eye view (top view) of the rough structure A, and Figs. 3(b), 4(b), 5(b), and 6(b) each are a perspective view of the protrusion constituting the rough structure A shown in Figs. 3(a), 4 (a), 5 (a), and 6 (a). As shown in Figs. 3(a) to 6(b), a rough structure A(7) is the total body of protrusions 8. That is, the membrane carrier 3 includes a flat portion 9 corresponding to the bottom surface of the flow path 2 of the liquid sample, and a plurality of protrusions 8 protruding from the flat portion 9. The first surface is a surface including the flat portion 9. The flat portion 9 (the first surface) does not have to be strictly flat. For example, the surface roughness of the flat portion 9 (the first surface) between the adjacent protrusions 8 can be made smaller than the surface roughness of the surface of the protrusions 8 (that is, the rough microstructure B) . Here, the surface roughness is, for example, an arithmetic mean roughness Ra or a root mean square roughness Rq calculated in the same manner as the above-described method of calculating Ra1, Ra2, Rq1, or Rq2, for example. By the capillary action, a space between the plurality of the protrusions 8 functions as a flow path 2 transporting the liquid sample along the surface of the membrane carrier 3. In other words, by the capillary action, a void in the rough structure A(7) functions as the flow path 2 transporting the liquid sample along the surface of the membrane carrier 3. The plurality of the protrusions 8 may be regularly or translationally symmetrically arranged on a surface (first surface) of the membrane carrier 3.

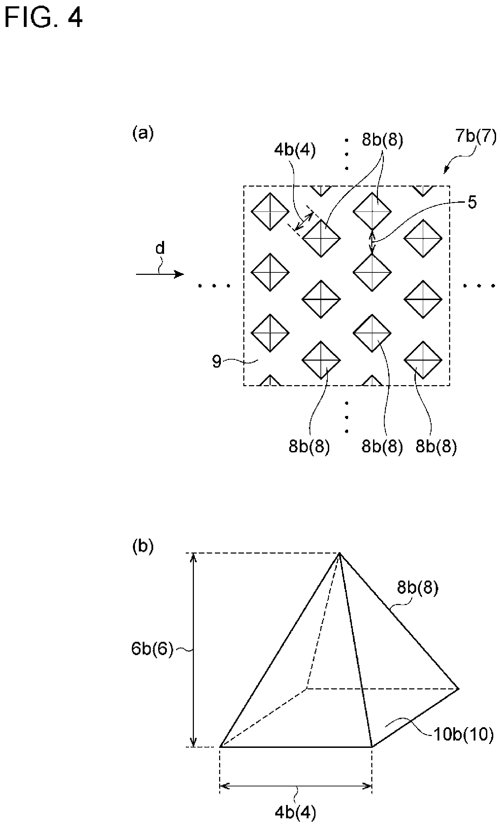

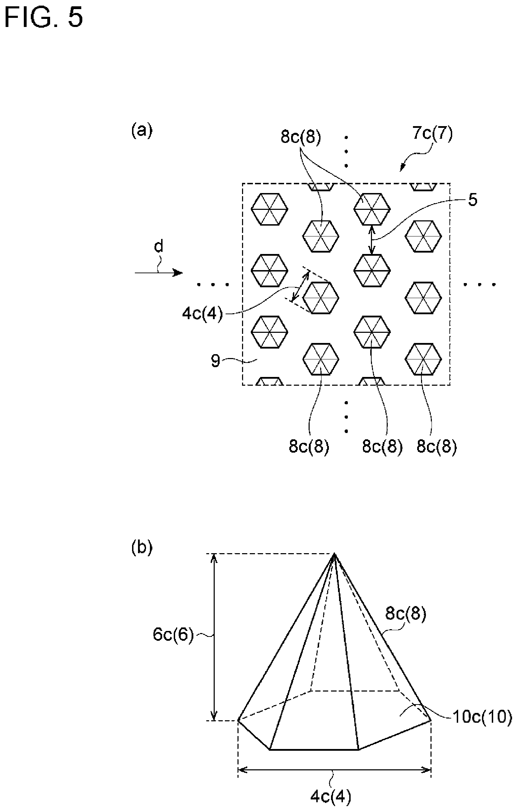

A shape of the plurality of the protrusions 8 constituting the rough structure A(7) can be freely selected. Examples of the shape of the protrusion 8 include a cone, a polygonal pyramid, a truncated cone, a truncated polypyramid, a cylinder, a polygonal prism, a hemisphere, a hemi-ellipsoid, and the like. Examples of a bottom surface of the rough structure A include a circle or a polygon (for example, a square, a rhombus, a rectangle, a triangle, a hexagon, and the like) . For example, as shown in Fig. 3, a shape of a protrusion 8a may be a cone. For example, as shown in Fig. 4, a shape of a protrusion 8b may be a quadrangular pyramid. For example, as shown in Fig. 5, a shape of a protrusion 8c may be a hexagonal pyramid. For example, as shown in Fig. 6, a shape of a protrusion 8d may be a quadrangular prism (a line and space structure in which the protrusion 8d is in a line shape) . From a viewpoint that the entire surface of the membrane carrier 3 can be visually recognized at a time when the rough structure A(7) is viewed from a bird's-eye view (viewed from above), and a change in color at a time when the substance to be detected has been detected is easily confirmed by an optical technique, among these, a conical body structure such as a cone or a polygonal pyramid is suitable as the shape of the protrusion 8. Among the conical body structures, cones are preferable.

The shape of the protrusion 8 constituting the rough structure A(7) does not need to be a geometrically accurate shape, and may be a shape with rounded corners or a shape with fine roughness on the surface.

A diameter 4 (average diameter) of the bottom surface 10 of the protrusion 8 constituting the rough structure A(7) is preferably equal to or more than 5 µm and equal to or less than 1000 µm, and more preferably equal to or more than 10 µm and equal to or less than 500 µm. In a case where the diameter 4 of the bottom surface 10 of the protrusion 8 is equal to or more than the lower limit value, the accuracy of fine processing can be suppressed low, and the cost for forming the rough structure A(7) tends to be low. In a case where the diameter 4 of the bottom surface 10 of the protrusion 8 is equal to or less than the upper limit value, the number of protrusions 8 in one test kit increases, and the liquid sample can be easily developed.

Here, for the diameter 4 of the bottom surface 10 of the protrusion 8, for example, it is possible to select five optional protrusions 8 from the rough structure A(7), and to employ an average value of the diameter of the bottom surface 10 of the selected five protrusions 8.

The diameter 4 of the bottom surface 10 of the protrusion 8 is defined as a representative length of the bottom surface 10 of the protrusion 8. The representative length of the bottom surface 10 is a diameter in a case where the shape of the bottom surface 10 is a circle, a shortest side length in a case where the shape of the bottom surface 10 is a triangle or a quadrangle, a longest diagonal length in a case where the shape of the bottom surface 10 is a polygon with more than four sides, and a longest length of the bottom surface 10 in a case where the shape of the bottom surface 10 is another shape.

As shown in Figs. 3(a) and 3(b), in a case where the shape of the protrusion 8a is a cone, the diameter 4a of the bottom surface 10a of the protrusion 8a is a diameter of the bottom surface (circle) of the cone. As shown in Figs. 4(a) and 4(b), in a case where the shape of the protrusion 8b is a regular quadrangular pyramid, the diameter 4b of the bottom surface 10b of the protrusion 8b is a length of a side of the bottom surface (regular square) 10b. As shown in Figs. 5(a) and 5(b), in a case where the shape of the protrusion 8c is a regular hexagonal pyramid, the diameter 4c of the bottom surface 10c of the protrusion 8c is the length of a diagonal line passing through the center of the bottom surface (regular hexagon) 10c (the length of the longest diagonal line). As shown in Figs. 6(a) and 6(b), in a case where the shape of the protrusion 8d is a rectangle, the diameter 4d of the bottom surface 10d of the protrusion 8d is a length of the shortest side of the bottom surface (rectangle) 10d (in Figs. 6(a) and 6(b), the length in a direction orthogonal to the transporting direction d of the liquid sample).

A height 6 (average height) (for example, height H1 and height H2, respectively) of the protrusion 8 constituting the rough structure A(7) is preferably equal to or more than 5 µm and equal to or less than 1,000 µm, and more preferably equal to or more than 10 µm and equal to or less than 500 µm. In a case where the height 6 of the protrusion 8 is equal to or more than the lower limit value, a volume of the flow path 2 increases, and the liquid sample can be developed in a shorter time. In a case where the height 6 of the protrusion 8 is equal to or less than the upper limit value, the time and cost for preparing the rough structure A(7) can be reduced, and the rough structure A(7) can be more easily prepared.

Here, for the height 6 of the protrusion 8, for example, it is possible to select five optional protrusions 8 from the rough structure A(7), and to employ an average value of the height of the five selected protrusions 8.

The height 6 of the protrusion 8 is defined as a maximum length of the protrusion 8 in a direction orthogonal to the flat portion 9. As shown in Figs. 3(a) and 3(b), in a case where the shape of the protrusion 8a is a cone, the height 6a of the protrusion 8a is the maximum length of the protrusion 8a in the direction orthogonal to the flat portion 9 (the height of the cone). As shown in Figs. 4(a) and 4(b), in a case where the shape of the protrusion 8b is a quadrangular pyramid, the height 6b of the protrusion 8b is the maximum length of the protrusion 8b in the direction orthogonal to the flat portion 9 (height of the quadrangular pyramid). As shown in Fig. 5(a) and 5(b), in a case where the shape of the protrusion 8c is a hexagonal pyramid, the height 6c of the protrusion 8c is the maximum length of the protrusion 8c in the direction orthogonal to the flat portion 9 (height of the hexagonal pyramid) . As shown in Fig. 6(a) and 6(b), in a case where the shape of the protrusion 8d is a quadrangular prism, the height 6d of the protrusion 8d is the maximum length of the protrusion 8d in the direction orthogonal to the flat portion 9 (the height of the quadrangular prism).

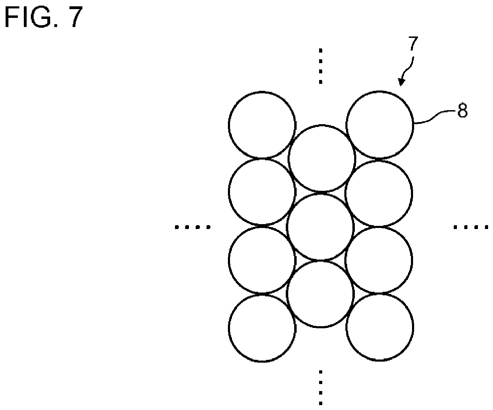

A distance 5 (average distance) between the adjacent protrusions in the rough structure A(7), that is, the closest contact distance between the protrusions 8 is preferably equal to or more than 0 and equal to or less than 500 µm. The upper limit of the closest contact distance is preferably 500 µm, more preferably 100 µm. The distance 5 between the adjacent protrusions cannot be less than 0 µm, and in a case where the distance 5 between the adjacent protrusions is equal to or less than the upper limit value, a contact area between the liquid sample and the flow path 2 increases, and with this, the capillary force increases. Therefore, it becomes easier to move the liquid sample. In particular, when the closest contact distance between the protrusions 8 is 0, for example, as shown in Fig. 7, the adjacent protrusions 8 are arranged without a gap, the number of protrusions 8 per unit area increases, and the detection signal can be enhanced. Here, the "distance between adjacent protrusions" is the closest contact distance between a pair of adjacent protrusions 8.

Here, for the distance 5 between the adjacent protrusions, for example, it is possible to select five distances between optional adjacent protrusions from the rough structure A(7), and to employ an average value of the distances between the five selected adjacent protrusions.

An aspect ratio of the protrusions 8 constituting the rough structure A(7) is preferably equal to or more than 0.1 and equal to or less than 10, and more preferably equal to or more than 0.1 and equal to or less than 2.0. The aspect ratio mentioned herein is a value (Lh/Lv) obtained by dividing the height 6 (Lh) of the protrusion 8 by the representative length (diameter 4) (Lv) of the bottom surface 10 of the protrusion 8. In a case where the aspect ratio is equal to or more than the lower limit value, the contact area between the liquid sample and the flow path 2 increases, and with this, the capillary force increases. Therefore, it becomes easier to move the liquid sample. In a case where the aspect ratio is equal to or less than the upper limit value, the rough structure A can be more easily prepared.

The rough structure A(7) and the membrane carrier 3 of a test kit 18 according to the present embodiment include a thermoplastic resin, for example. In other words, by processing a membrane-shaped base material formed of a thermoplastic resin, it is possible to prepare a membrane carrier 3 having a rough structure A(7).

Examples of a processing method include thermal imprinting, UV imprinting, injection molding, etching, photolithography, mechanical cutting, laser processing, and the like. Among these, as a technique of performing accurate processing at a low cost, thermal imprinting on a thermoplastic resin is suitable.

Examples of the thermoplastic resin include a polyester-based resin, a polyolefin-based resin, a polystyrene-based resin, a polycarbonate-based resin, a fluorine-based resin, a (meth)acrylic-based resin, and the like, and specifically, various kinds such polyethylene terephthalate (PET), cycloolefin polymer (COP), polypropylene (PP), polystyrene (PS), polycarbonate (PC), polyvinylidene fluoride (PVDF), polymethyl methacrylate (PMMA), polyethylene (PE), and the like can be used. These thermoplastic resins may be used alone, or may be used in combination of two or more.

In a case of a processing method using a mold such as imprinting or injection molding, the conical body is thinner at the top than at the bottom. Therefore, a volume to be carved out during mold preparation is smaller than that of a column preparation with the same bottom surface, and thus it is possible to prepare a mold at a low cost. In this case, it is possible to perform detection of the substance to be detected in the liquid sample at a lower cost.

As described above, the membrane carrier 3 includes a rough structure A(7) provided on one surface of the membrane carrier 3, a flow path 2 formed by the rough structure A(7) and for transporting a liquid sample, and a detection zone (detection portion) 3y for detecting the substance to be detected in the liquid sample. The membrane carrier 3 may be the membrane carrier 3 for the test kit 18 for detecting the substance to be detected in the liquid sample.

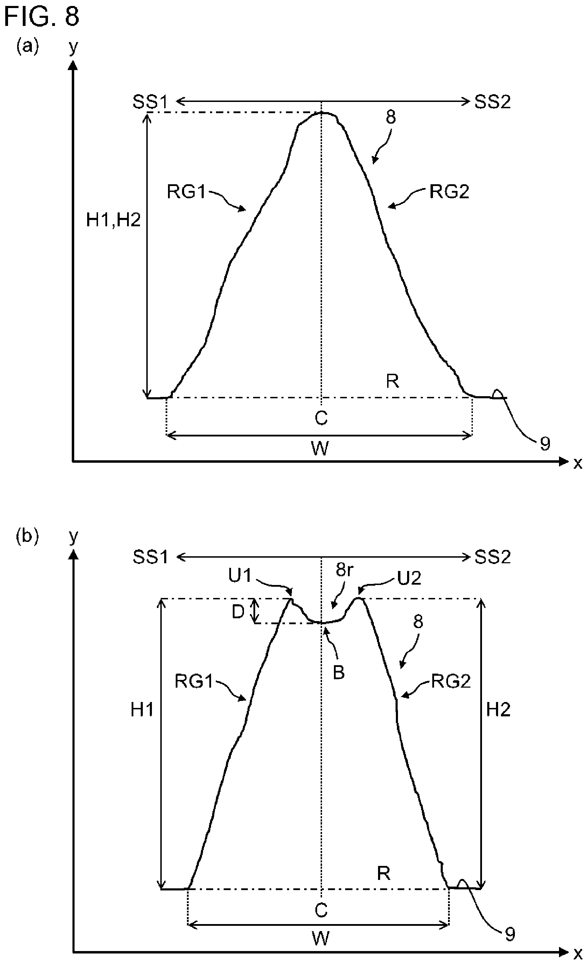

Fig. 8 (a) is a diagram for explaining a first example of a method of measuring various parameters of the protrusion 8 on which the rough microstructure B is formed in the rough structure A(7). Fig. 8(b) is a diagram for explaining a second example of a method of measuring various parameters of the protrusion 8 on which the rough microstructure B is formed in the rough structure A(7). In Figs. 8(a) and 8(b), an x direction indicates a width direction of the protrusion 8, and a y direction indicates a height direction of the protrusion 8.

First, Fig. 8(a) is described.

The membrane carrier 3 is cut along a straight line passing through a center of the protrusion 8 when viewed from an upper surface of the membrane carrier 3, and the cut cross-section of the membrane carrier 3 is observed with a scanning electron microscope (SEM) to obtain a cross-sectional SEM image of the protrusion 8. The cross-section of the protrusion 8 can be obtained, for example, by cutting with a focused ion beam (FIB), a microtome, or the like. The cross-sectional SEM image of the protrusion 8 is analyzed by an image analysis apparatus (for example, a computer on which image analysis software is installed). The cross-sectional SEM image of the protrusion 8 in the cross-sectional SEM image is analyzed by an image analysis apparatus to obtain a fitting curve (for example, a Gaussian distribution curve) for the outer edge of the protrusion 8. The fitting curve has a line-symmetrical shape, and a center line C of the protrusion 8 is positioned on an axis of symmetry of the fitting curve.

The protrusion 8 has a first region RG1 and a second region RG2. The first region RG1 of the protrusion 8 is positioned on one side SS1 (left side in example shown in Fig. 8(a)) of the protrusion 8 from the center line C of the protrusion 8 in a width direction (x direction) of the protrusion 8. The second region RG2 of the protrusion 8 is positioned on the other side SS2 (in an example shown in Fig. 8(a), right side) of the protrusion 8 from the center line C of the protrusion 8 in the width direction (x direction) of the protrusion 8.

The protrusion 8 has a width W. The width W of the protrusion 8 is a distance between a position of a left end of the first region RG1 and a position of a right end of the second region RG2. The position of the left end of the first region RG1 and the position of the right end of the second region RG2 are intersections of a reference line R orthogonal to the center line C of the protrusion 8 and positioned at a height 0 of the protrusion 8, and an outer edge of the protrusion 8.

The first region RG1 of the protrusion 8 has a height H1 in the height direction (y direction) of the protrusion 8, and the second region RG2 of the protrusion 8 has a height H2 in the height direction (y direction) of the protrusion 8. The height H1 of the first region RG1 is a maximum height of the protrusion 8 in the first region RG1, and the height H2 of the second region RG1 is a maximum height of the protrusion 8 in the second region RG2. For example, in a case where the protrusion 8 has the maximum height at the center line C (for example, Fig. 8(a)), the height H1 of the first region RG1 and the height H2 of the second region RG1 are equal to each other. On the other hand, for example, in a case where the protrusion 8 has a maximum height at a position deviated from the center line C to one side SS1, the height H1 of the first region RG1 is higher than the height H2 of the second region RG1. The heights H1 and H2 of the protrusion 8 are calculated by analyzing the cross-sectional SEM image of the protrusion 8 with an image analysis apparatus.

The protrusion 8 has an arithmetic mean roughness Ra1 and a root mean square roughness Rq1 for at least a part of the outer edge of the first region RG1. Ra1 and Rq1 are calculated by analyzing the cross-sectional SEM image of the protrusion 8 with an image analysis apparatus according to the above-described method.

The protrusion 8 has an arithmetic mean roughness Ra2 and a root mean square roughness Rq2 for at least a part of the outer edge of the second region RG2. Ra2 and Rq2 are calculated by analyzing the cross-sectional SEM image of the protrusion 8 with an image analysis apparatus according to the above-described method.

Subsequently, Fig. 8(b) is described.

A concave portion 8r having a depth D is formed at the tip of the protrusion 8. The depth D is equal to or more than 1.0 µm. The depth D of the concave portion 8r is a distance between a lowermost end B of the concave portion 8r and a lower one of two upper ends U1 and U2 of the protrusion 8 in the height direction (y direction) of the protrusion 8. The upper end U1 of the protrusion 8 is positioned on one side SS1 with respect to the center line C of the protrusion 8. The upper end U2 of the protrusion 8 is positioned on the other side SS2 with respect to the center line C of the protrusion 8.

The first region RG1 is a region excluding the concave portion 8r. Therefore, the outer edge of the protrusion 8 in the first region RG1 includes only a portion that is macroscopically inclined upward toward the center line C of the protrusion 8.

The second region RG2 is a region excluding the concave portion 8r. Therefore, the outer edge of the protrusion 8 in the second region RG2 includes only a portion that is macroscopically inclined upward toward the center line C of the protrusion 8.

The protrusion 8 has a width W. The width W of the protrusion 8 is a distance between a position of a left end of the first region RG1 and a position of a right end of the second region RG2. A position of the left end of the first region RG1 and a position of the right end of the second region RG2 are determined in the same manner as in the method described with reference to Fig. 8(a).

The number of concave portions R in the protrusion 8 on which the rough microstructure B is formed can be adjusted within the numerical value range by adjusting the mold when preparing the membrane carrier 3 having the rough structure A(7) by thermal imprinting. In particular, it is preferable to adjust the number of concave portions R in the protrusion 8 on which the rough microstructure B is formed by adjusting a shape of a mold surface used for thermal imprinting. For example, it is preferable to adjust the number of concave portions R in the protrusion 8 on which the rough microstructure B is formed by laser processing a surface of the mold. In laser processing, the mold can be irradiated with a pulse laser a plurality of times. The pulse laser is preferably an ultrashort pulse laser, such as a femtosecond laser. By shortening a pulse width of the pulse laser, specifically, for example, by setting the pulse width to be equal to or less than the femtosecond order, the rough structure formed on the surface of the mold can be made finer, and with this, the number of concave portions R in the protrusion 8 can be made large.

That is, a method of manufacturing a test kit 18 according to the present embodiment preferably includes a step (thermal imprinting step) of preparing a membrane carrier 3 having the rough structure A(7) by thermal imprinting. In the thermal imprinting step, the surface of the mold on which a plurality of concave portions are formed is applied to a membrane-shaped base material made of a thermoplastic resin, and the base material is heated to form a membrane carrier 3 having the rough structure A(7) (a plurality of protrusions 8) corresponding to the shape of the concave portion and the flat portion 9.

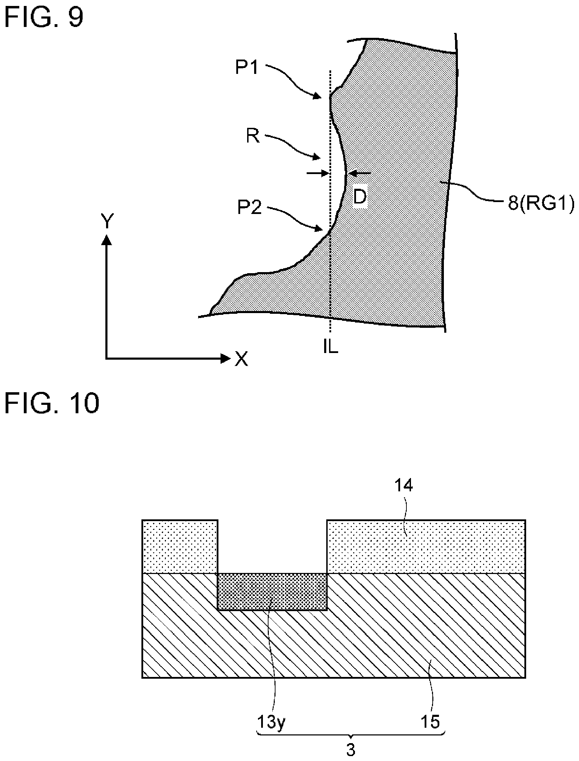

Fig. 9 is a diagram for explaining a concave portion R of the outer edge in the first region RG1 of the protrusion 8. Fig. 9 corresponds to an enlarged view of the outer edge of the protrusion 8 shown in Fig. 8 (a) or Fig. 8(b). In Fig. 9, the X direction indicates a direction parallel to the flat portion 9 (the first surface) . The Y direction indicates a direction perpendicular to the flat portion 9 (the first surface).

The outer edge of the first region RG1 of the protrusion 8 has a concave portion R. The concave portion R is recessed in the X direction by a distance D with respect to a virtual straight line IL. The distance D is equal to or more than 50 nm and equal to or less than 500 nm. The virtual straight line IL extends in the Y direction. Specifically, the virtual straight line IL is in contact with a first position P1 of the outer edge of the protrusion 8 and passes through a second position P2. The first position P1 of the outer edge of the protrusion 8 is a position positioned on an outermost side of upper end portions of the concave portion R of the protrusion 8. Therefore, the virtual straight line IL is in contact with the first position P1. That is, in the first position P1 and the vicinity thereof, the virtual straight line IL and the outer edge of the protrusion 8 geometrically share only the first position P1 (that is, one point) . A first position P2 of the outer edge of the protrusion 8 is positioned below the first position P1 in the Y direction.

Fig. 9 shows one concave portion R in a part of the outer edge in the first region RG1 of the protrusion 8. As described above, in the embodiment, the number of the concave portions R in the entire first region RG1 is not limited to one, but is equal to or more than a certain number.

The concave portion of the outer edge in the second region RG2 of the protrusion 8 is also determined in the same manner as in the method shown in Fig. 9.

In an embodiment, at least one of a carbon atom and a nitrogen atom, and an oxygen atom may be present on the surface of the detection zone.

The ratio of the number of oxygen atoms to the total number of atoms of each atom (number of oxygen atoms/(number of carbon atoms + number of nitrogen atoms + number of oxygen atoms)) is equal to or more than 0.01 and equal to or less than 0.50. In the membrane carrier of one embodiment, the oxygen atomic number ratio (number of oxygen atoms/(number of carbon atoms + number of nitrogen atoms + number of oxygen atoms)) on the surface of the detection zone is equal to or more than 0.01, preferably equal to or more than 0.05, more preferably equal to or more than 0.10, and further more preferably equal to or more than 0.20. In the membrane carrier of one embodiment, the oxygen atomic number ratio (number of oxygen atoms/(number of carbon atoms + number of nitrogen atoms + number of oxygen atoms)) on the surface of the detection zone is equal to or less than 0.50, preferably equal to or less than 0.40, more preferably equal to or less than 0.38, further more preferably equal to or less than 0.36, further more preferably equal to or less than 0.30, and further more preferably equal to or less than 0.10. The increase of the oxygen atomic number ratio on the surface of the detection zone makes it easier for the detection substance to adhere to the surface. As the detection substance adheres to the surface, the amount of the detection substance that is washed away when the liquid sample is developed is reduced, and highly sensitive test becomes possible. In a case where the oxygen atomic number ratio on the surface of the detection zone is equal to or less than the upper limit value, the occurrence of erroneous detection due to the reaction between the labeling substance and the detection substance when a solution not containing a substance to be detected is developed is further suppressed.

The oxygen atomic number ratio on the surface of the detection zone is calculated by X-ray photoelectron spectroscopy (XPS). The calculation of the oxygen atomic number ratio by XPS is described below. A binding energy correction of the spectrum obtained by the measurement is performed by a C-C bond in a C1s spectrum. A background (BG) is subtracted for each peak of the C1s spectrum, an N1s spectrum, and an O1s spectrum of the spectrum subjected to the binding energy correction. A peak area (signal intensity) of each atom calculated by subtracting the BG from each peak is divided by a correction coefficient (relative sensitivity coefficient, transmission function, and kinetic energy correction) and is calculated such that the total area after correction becomes 100. Each of the obtained values is defined as the number of carbon atoms, the number of nitrogen atoms, and the number of oxygen atoms, and the oxygen atomic number ratio (the number of oxygen atoms/(the number of carbon atoms + the number of nitrogen atoms + the number of oxygen atoms)) is calculated.

The oxygen atomic number ratio on the surface of the detection zone can be adjusted within the range by surface-treating the surface of the detection zone. The surface treatment method is not limited at all, and various techniques such as various plasma treatments, corona treatments, UV irradiation, and surface modification by UV/ozone treatment can be used.

It is preferable that the surface treatment is performed only in the detection zone. By performing the method only in the detection zone, the detection substance does not adhere to a non-detection zone (region other than the detection zone) in the flow path, and the detection substance can adhere only to the detection zone with high efficiency. As a result, a detection signal can be easily recognized in the detection zone (the S/N ratio becomes high).

Examples of a method of selectively surface-treating the surface of the detection zone to modify the surface of the detection zone include a method of covering sites other than the detection zone with a shieldable mask (shield) and performing surface treatment on the exposed detection zone. Fig. 10 is a diagram for explaining a method of selectively surface-treating the surface of the detection zone. A shield 14 having a void portion is disposed on the membrane carrier 3 to expose the detection zone (surface treatment portion). Aportion of the membrane carrier 3 covered with the shield 14 becomes the untreated portion (non-detection zone) 15. As the shield 14, a metal plate is preferable. By surface-treating the exposed site, the membrane carrier 3 having an oxygen atomic number ratio on the surface of the detection zone within the range is obtained.

In the embodiment, as the material of the membrane carrier, it is preferable to use a resin having a surface oxygen atomic number ratio (number of oxygen atoms/(number of carbon atoms + number of nitrogen atoms + number of oxygen atoms)) of less than 0.01, and it is more preferable to use a resin having a surface oxygen atomic number ratio of equal to or less than 0.005. The resin having an oxygen atomic number ratio on the surface of less than 0.01 is a resin not containing oxygen atoms in the structural formula of the main component, and may be a resin containing carbon atoms and not containing nitrogen atoms and oxygen atoms, such as a polyolefin-based resin, a polystyrene-based resin, a fluorine-based resin, and the like. Specific examples of such a resin include polyethylene (PE), cycloolefin polymer (COP), polypropylene (PP), polystyrene (PS), polyvinylidene fluoride (PVDF), and the like. The resin having an oxygen atomic number ratio on the surface of less than 0.01 may be a resin containing carbon atoms and nitrogen atoms and not containing oxygen atoms such as a polyimide resin. In a case where a resin containing carbon atoms and not containing nitrogen atoms and oxygen atoms is used, the oxygen atomic number ratio (number of oxygen atoms/(number of carbon atoms + number of nitrogen atoms + number of oxygen atoms)) in the detection zone is substantially equal to a value of the number of oxygen atoms/ (number of carbon atoms + number of oxygen atoms).

In a case where the oxygen atomic number ratio on the surface is equal to or less than 0.005, when a membrane carrier is prepared, a test kit is prepared using the prepared membrane carrier, and a liquid sample is developed, attachment of a labeling substance to a non-detection zone is further suppressed. In a case where the labeling substance is attached to the non-detection zone, even if a signal having the same intensity is generated in the detection zone, it becomes difficult to recognize thereof (the S / N ratio becomes low) .

In a case where the oxygen atomic number ratio on the surface is equal to or less than 0.005, when a membrane carrier is prepared, a test kit is prepared using the prepared membrane carrier, and a liquid sample is developed, attachment of a labeling substance to a non-detection zone is further suppressed. In a case where the labeling substance is attached to the non-detection zone, even if a signal having the same intensity is generated in the detection zone, it becomes difficult to recognize thereof (the S / N ratio becomes low) .

In the test kit 18 according to the present embodiment, the detection zone 3y of the membrane carrier 3 shows a change in color when the substance to be detected is detected. The change in color may be a change in color that can be confirmed by an optical technique.

Examples of the optical technique mainly include two techniques of visual determination and measurement of fluorescence/emission intensity. In a case of visual determination, a change in color preferably occurs such that color difference between two color stimuli at a time of measuring color before and after the detection by a color system of the CIE1976L*a*b* color space (ΔE described in JIS Z8781-4: 2013) is equal to or more than 0.5. In a case where the color difference is equal to or more than 0.5, it is easy to visually confirm the color difference. In a case where the fluorescence/emission intensity is measured and determined, the change in color preferably occurs such that a ratio (FI1/FI2) of fluorescence/emission intensity (FI1) in the detection zone 3y and fluorescence/emission intensity (FI2) in an upstream area and a downstream area adjacent to the detection zone 3y = equal to or more than 10/1. In a case where the ratio is equal to or more than 10/1, separation of signal and noise becomes easy.

In order to prepare the detection zone 3y in the test kit 18 of the present embodiment, in an embodiment, a detection substance is immobilized on at least a part of the flow path 2. That is, the detection substance for detecting a substance to be detected is immobilized in the detection zone 3y. The change in color in the detection zone 3y is caused by holding the substance to be detected in the detection zone 3y by the detection substance (reacting with the detection substance).

In other words, the method of manufacturing the test kit 18 includes a step of immobilizing the detection substance in the detection zone 3y, which causes a change in color by holding the substance to be detected in the detection zone 3y. From a viewpoint that the detection substance (reagent) can be more efficiently immobilized in the detection zone 3y, pre-surface treatment may be performed in a site provided with the detection zone 3y in the membrane carrier 3. As the surface treatment method, the method exemplified above can be used.

In the present embodiment, examples of the detection substance (reagent) include an antibody. The antibody is an antibody that reacts with a substance to be detected by an antigen-antibody reaction, and may be a polyclonal antibody or a monoclonal antibody.

The change in color in the detection zone 3y may be caused by a label having an antibody or an antigen-binding fragment thereof that specifically reacts with the substance to be detected in the liquid sample. The change in color occurs as the label is held in the detection zone 3y by the detection substance (reacts (binds) with the detection substance) and colored, for example.

The label may be a labeled antibody such as a fluorescently labeled antibody, a chemiluminescently labeled antibody, or an enzyme-labeled antibody, and may be one in which the antibody or the antigen-binding fragment thereof binds to a particle such as colloidal particles and latex particles. The antigen-binding fragment refers to a fragment that can specifically bind to a substance to be detected, for example, an antigen-binding fragment of an antibody. The label can bind to the substance to be detected through an antibody or an antigen-binding fragment thereof. The particles may have magnetism or fluorescent properties. Examples of the colloidal particles include metal colloidal particles such as gold colloidal particles and platinum colloidal particles. The particles are preferably latex particles in view of particle diameter control, dispersion stability, and ease of binding. Although the material of the latex particles is not particularly limited, polystyrene is preferable.

The particles are preferably colored particles or fluorescent particles, and more preferably colored particles in view of visibility. The colored particles may be any particles as long as the color can be detected with naked eye. The fluorescent particles may contain a fluorescent substance. The particles may be colored latex particles or fluorescent latex particles. In a case where the particles are colored latex particles, the change in color described above is suitably determined visually. In addition, in a case where the particles are fluorescent latex particles, the change in color described above is suitably determined by measuring the fluorescence intensity.

Such label described above is provided in at least a part of the test kit 18 so as to react with the substance to be detected in the dropped liquid sample. The label may be provided in a member in the test kit 18 or may be provided in at least a part (upstream side from the detection zone 3y) of the flow path 2 of the membrane carrier 3. The label that has reacted (bound) with the substance to be detected is held in the detection zone 3y by the detection substance (by the detection substance reacting (binding) with the substance to be detected). With this, a change in color (color development by the label) occurs in the detection zone 3y.

The test method of the liquid sample according to an aspect of the present embodiment is a test method using the test kit 18.

A test method of a liquid sample using the test kit 18 may include a step of mixing a liquid sample and a label specifically binding to a substance to be detected in the liquid sample, preparing a mixture solution sample (mixed liquid sample), and binding the substance to be detected and the label to each other; a step of dropping the mixture solution sample onto a dropping zone 3x provided in the membrane carrier 3; a step of transporting the mixture solution sample from the dropping zone 3x to the detection zone 3y by the rough structure A(7); and a step of detecting a change in color (color development by the label) in the detection zone 3y.

In addition, for example, the test method may include dropping the liquid sample onto the dropping zone 3x on the surface of the membrane carrier 3; transporting the liquid sample from the dropping zone 3x to the detection zone 3y through the rough structure A(7) by the capillary action by the rough structure A(7) (a plurality of protrusions 8) formed on the surface of the membrane carrier 3; and detecting a change in color in the detection zone 3y (optically determining the presence or absence of a change in color) by binding the substance to be detected in the liquid sample to a label through the antibody or antigen-binding fragment thereof, and binding the substance to be detected to a reagent immobilized in the detection zone 3y, in the transporting process.

In binding the substance to be detected and the label to each other of the test method, the method of mixing the liquid sample with the label is not particularly limited. For example, the method may be a method of adding a liquid sample to a container containing the label, for example, or may be a method of mixing a liquid containing the label with the liquid sample. In addition, for example, a filter may be interposed between dropping ports of the container containing the liquid sample, and the label may be immobilized in the filter.

As described above, although the embodiments of the present invention have been described above with reference to the drawings, these are examples of the present invention, and various configurations other than the above can be employed.

### Examples

Hereinafter, the present embodiment will be specifically described with reference to examples and comparative examples, but the present embodiment is not limited to these examples.

### [Example 1]

### <Preparation of membrane carrier>

A polycarbonate sheet (manufactured by Teijin Ltd., film thickness 200 µm) was thermally imprinted, and a membrane carrier was prepared such that a conical-shaped protrusion 8 having a diameter of a bottom surface of a rough structure A (protrusion) (hereinafter, also referred to as "diameter of the protrusion" or "diameter") of 30 µm and a height of the rough structure A (protrusion) (hereinafter, also referred to as "height") of 30 µm was arranged in a triangular array format as shown in Fig. 7 with an average distance between centers of the protrusions of 30 µm. That is, vertically adjacent protrusions 8 in Fig. 7 are arranged without a gap, and diagonally adjacent protrusions 8 in Fig. 7 are arranged without a gap (the distance 5 shown in Fig. 3 becomes 0).

Here, when performing thermal imprinting, a laser-processed mold was used to form a rough microstructure B on the surface of the rough structure A (protrusion), and the number of concave portions R on the outer edge in the first region RG1 and the number of concave portions R on the outer edge of the second region RG2 of the protrusion 8 on which the rough microstructure B is formed were adjusted, respectively, to the values shown in Table 1.

The types of molds used are as follows.

A nickel mold in which conical-shaped holes having a diameter of 30 µm at the entrance of the holes and a depth of 30 µm are arranged in a triangular array format with an average distance between centers of the holes of 30 µm was irradiated with pulse light a plurality of times from a laser processing apparatus (ultrashort pulse laser processor R-200 manufactured by Tosei Electrobeam, laser wavelength: 1552 nm, rated output: 10 W, pulse: femtosecond) to obtain a mold.

As shown in Fig. 8(a), a macroscopically large concave portion (for example, the concave portion 8r shown in Fig. 8(b)) was not formed at the tip of the protrusion 8. The number of concave portions R on the outer edge in the first region RG1 and the number of concave portions R on the outer edge in the second region RG2 of the protrusion 8 were measured using a computer in which image analysis software (ImageJ) was installed as an image analysis apparatus (refer to Figs. 8(a) and 9) .

In addition, using a computer in which image analysis software (MATLAB (registered trademark) manufactured by MathWorks) is installed as an image analysis apparatus, according to the method described in the implementation method, Ra1 and Rq1 were calculated for the outer edge of the first region RG1 of the protrusion 8, and Ra2 and Rq2 were calculated for the outer edge of the second region RG2 of the protrusion 8 according to the method described in the embodiment. Ra1 and Rq1 were calculated from the outer edge of a portion of the first region RG1 from the height of 1 µm from the flat portion 9 to the height of 25 µm from the height, and Rq2 and Ra2 were calculated from the outer edge of a portion of the second region RG2 from the height of 1 µm from the flat portion 9 to the height of 25 µm from the height.

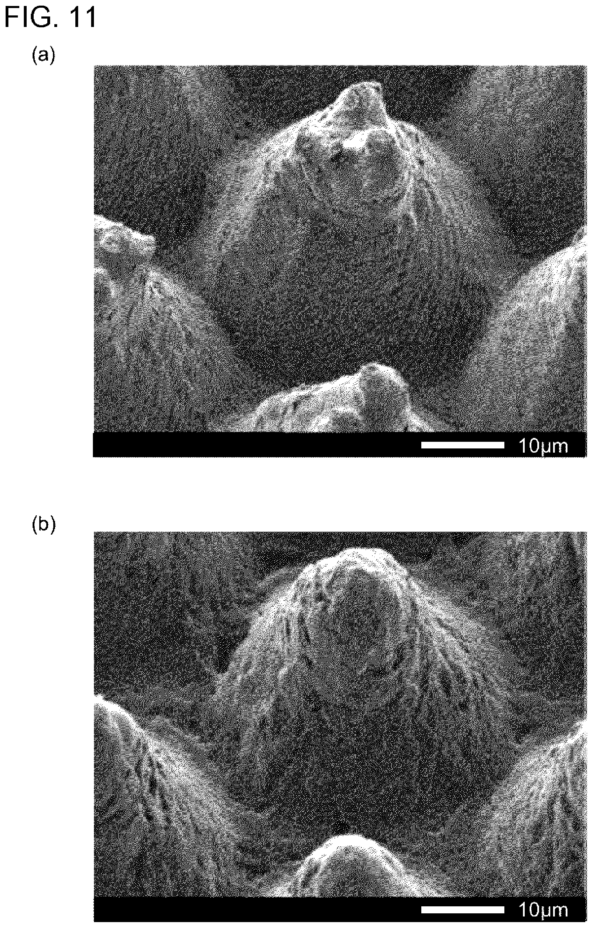

Fig. 11(a) is a diagram showing an electron micrograph showing an enlarged view of the protrusion 8 according to Example 1.

### <Measurement of amount of antibody carried>

An anti-CRP antibody solution having a concentration of 0.1 mg/mL was prepared by diluting a fluorescently labeled anti-CRP (C-reactive protein) antibody with a buffer solution (composition: 50 mM tris buffer solution pH 7.5, trehalose 2 (w/v)%), and applied to the membrane carrier 3 in an amount of 1 µL. After performing drying at 45°C for 1 hour, ultrasonic cleaning was performed in a Triton X-100 (product name) 2 (v/v) % aqueous solution. After drying at room temperature, the fluorescence intensity of the remaining antibody was evaluated using a fluorescence microscope (BZ-X710 manufactured by KEYENCE Corporation).

### [Example 2]

Example 2 was the same as that of Example 1 except for the type of the mold.

The types of molds used are as follows.

An aluminum mold in which conical-shaped holes having a diameter of 30 µm at the entrance of the holes and a depth of 30 µm are arranged in a triangular array format with an average distance between centers of the holes of 30 µm was irradiated with pulse light a plurality of times from a laser processing apparatus (an ultrashort pulse laser processor R-200 manufactured by Tosei Electrobeam, laser wavelength: 1552 nm, rated output: 10 W, pulse: femtosecond) to obtain a mold.

As shown in Fig. 8(a), a macroscopically large concave portion (for example, the concave portion 8r shown in Fig. 8(b)) was not formed at the tip of the protrusion 8. The obtained results are shown in Table 1. Each parameter was measured by the method described with reference to Fig. 8(a).

Fig. 11(b) is a diagram showing an electron micrograph showing an enlarged view of the protrusion 8 according to Example 2.

### [Comparative Example]

The comparative example was the same as that of Example 1 except for the type of the mold.

The types of molds used are as follows.

A nickel mold in which conical-shaped holes having a diameter of 30 µm at the entrance of the holes and a depth of 30 µm are arranged in a triangular array format with an average distance between centers of the holes of 30 µm was cut (by not using the laser processing apparatus used in Examples 1 and 2) to obtain a mold.

As shown in Fig. 8(b), a macroscopically large concave portion 8r was formed at the tip of the protrusion 8. The obtained results are shown in Table 1. Each parameter was measured by the method described with reference to Fig 8(b).

**[Table 1]**

| | Example 1 | Example 2 | Comparative Example |
|---|---|---|---|
| Number of concave portions in first region | 15 | 3 | 1 |
| Number of concave portions in second region | 13 | 4 | 1 |
| Arithmetic mean roughness Ra1 [µm] | 0.027 | 0.023 | 0.009 |
| Root mean square roughness Rq1 [µm] | 0.033 | 0.035 | 0.019 |
| Arithmetic mean roughness Ra2 [µm] | 0.025 | 0.024 | 0.011 |
| Root mean square roughness Rq2 [µm] | 0.032 | 0.036 | 0.021 |
| Fluorescence intensity [a.u.] | 71.8 | 45.8 | 36.5 |

From the results in Table 1, it was shown that the membrane carrier for a test kit according to the present embodiment can increase the amount of antibody carried as a detection substance as the number of concave portions R in the first region RG1 or the second region RG2 is equal to or more than a certain number and can detect the substance to be detected with high sensitivity.

This application claims priority based on Japanese Patent Application No. 2019-092042, filed on May 15, 2019, the disclosure of which is incorporated by reference in its entirety.

### REFERENCE SIGNS LIST

2: flow path
3: membrane carrier
3x: dropping zone
3y: detection zone (detection portion)
4, 4a, 4b, 4c, 4d: representative length on the bottom surface of the protrusion (diameter of the bottom surface of the protrusion)
5: distance between adjacent protrusions
6, 6a, 6b, 6c, 6d: height of protrusion
7, 7a, 7b, 7c, 7d: rough structure A
8, 8a, 8b, 8c, 8d: protrusion
8r: concave portion
9: flat portion
10, 10a, 10b, 10c, 10d: bottom surface of protrusion
13y: detection zone
14: shield
15: untreated portion
18: test kit
18a: housing
18b: first opening
18c: second opening
19: center of protrusion
20: straight line passing through the center of the protrusion
20d: length of a straight line passing through the center of the protrusion
d: flow direction of liquid sample (transporting direction)
B: lowermost end
C: center line
IL: virtual straight line
P1: first position
P2: second position
R: reference line
RG1: first region
RG2: second region
U1: upper end
U2: upper end

## Claims

1. A membrane carrier comprising:
a detection zone having a first surface on which a protrusion is formed,
wherein in one cross-section, the protrusion includes a first region positioned on one side of the protrusion from a center of the protrusion in a width direction of the protrusion, and a second region positioned on other side of the protrusion from the center of the protrusion in the width direction of the protrusion, and
in the one cross-section, at least one of an outer edge of the first region and an outer edge of the second region of the protrusion has a concave portion.

2. The membrane carrier according to Claim 1,
wherein in the one cross-section, the at least one of the outer edge of the first region and the outer edge of the second region of the protrusion has a plurality of concave portions recessed by equal to or more than 50 nm and equal to or less than 500 nm in a direction parallel to the first surface with respect to a virtual straight line being in contact with a first position of the outer edge and passing through a second position of the outer edge positioned below the first position in a direction perpendicular to the first surface.

3. The membrane carrier according to Claim 1 or 2,
wherein an arithmetic mean roughness Ra1 of at least a part of the outer edge of the protrusion in the first region calculated by a method including the following steps or an arithmetic mean roughness Ra2 of at least a part of the outer edge of the protrusion in the second region calculated by the method including the following steps is equal to or more than 0.020 µm and equal to or less than 1.000
µm, (Step 1) Extracting a profile of the at least a part of the outer edge
(Step 2) Trend removing the profile so that a start point and an end point of the profile overlap with a fitting curve of the profile
(Step 3) Calculating Ra1 or Ra2 by applying 1 µm based on a cutoff value λc defined in JIS B0601: 2013, without applying a cutoff value λs defined in JIS B0601: 2013, to the trend-removed profile.

4. The membrane carrier according to any one of Claims 1 to 3,
wherein a root mean square roughness Rq1 of at least a part of the outer edge in the first region of the protrusion calculated by a method including the following steps or a root mean square roughness Rq2 of at least a part of the outer edge in the second region of the protrusion calculated by the method including the following steps is equal to or more than 0.030 µm and equal to or less than 1.000
µm, (Step 1) Extracting a profile of the at least a part of the outer edge
(Step 2) Trend removing the profile so that a start point and an end point of the profile overlap with a fitting curve of the profile
(Step 3) Calculating Rq1 or Rq2 by applying 1 µm based on a cutoff value λc defined in JIS B0601: 2013, without applying a cutoff value λs defined in JIS B0601: 2013, to the trend-removed profile.

5. The membrane carrier according to any one of Claims 1 to 4,
wherein the first surface has a plurality of protrusions regularly or translationally symmetrically arranged and including the protrusion.

6. The membrane carrier according to Claim 5,
wherein a surface roughness of the first surface between adjacent protrusions is smaller than a surface roughness of a surface of the protrusion.

7. The membrane carrier according to Claim 5 or 6,
wherein a closest contact distance between adjacent protrusions is equal to or more than 0 and equal to or less than 500 µm.

8. The membrane carrier according to any one of Claims 1 to 7,
wherein the protrusion includes a thermoplastic resin.

9. The membrane carrier according to any one of Claims 1 to 8,
wherein a surfactant is attached to the protrusion.

10. A test kit comprising:
the membrane carrier according to any one of Claims 1 to 9.
